(19) Europäisches Patentamt
European Patent Office
Office européen des brevets

(11) **EP 1 291 024 A1**

(12) **EUROPÄISCHE PATENTANMELDUNG**

(43) Veröffentlichungstag:
**12.03.2003 Patentblatt 2003/11**

(51) Int Cl.[7]: **A61L 15/26**, A61F 13/15

(21) Anmeldenummer: **02019830.5**

(22) Anmeldetag: **06.09.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **11.09.2001 DE 10144713**

(71) Anmelder: **Sandler AG**
**95126 Schwarzenbach/Saale (DE)**

(72) Erfinder:
- **Höflich, Wolfgang**
  **95126 Schwarzenbach/S. (DE)**
- **Bernhuber, Uwe**
  **95028 Hof (DE)**

(54) **Permanent hydrophile Flüssigkeitstransportlage**

(57) Die Entwicklung im Bereich der Hygieneartikel, namentlich Windeln, Slipeinlagen, aber auch Produkte für den Inkontinenzbereich macht es erforderlich, für eine wiederholt rasche Flüssigkeitsaufnahme und -weiterleitung dauerhaft hydrophile Flüssigkeitstransportlagen einzusetzen. Um dies zu gewährleisten zeigt die Erfindung die Verwendung einer partiell oxifluorierten, aus thermoplastischen Polymeren bestehenden Schicht als permanent hydrophile Flüssigkeitstransportlage in Hygieneprodukten. Sie ist gekennzeichnet durch einen Gehalt an Fluor, der 25 Atomprozent nicht übersteigt.

EP 1 291 024 A1

## Beschreibung

Die Entwicklung im Bereich der Hygieneartikel, namentlich Windeln, Slipeinlagen, aber auch Produkte für den Inkontinenzbereich macht es erforderlich, für eine wiederholt rasche Flüssigkeitsaufnahme und -weiterleitung dauerhaft hydrophile Flüssigkeitstransportlagen einzusetzen.

Handelsübliche Hygieneartikel haben dabei den folgenden, schematischen Aufbau:

- dem Benutzer zugewandte Hüll-Lage aus Vlies und/oder Folie
- Aufnahme-/ Verteillage aus beispielsweise Vlies
- Saug/Speicherschicht aus beispielsweise Vlies mit superabsorbierenden Substanzen
- dem Benutzer abgewandte Wäscheschutzschicht aus Vlies oder Folie

Der Begriff Flüssigkeitstransportlage wird nachfolgend synonym für eine der oben genannten Schichten verwendet, er kann also sowohl für Hüll-Lagen aus Vlies und/oder Folie, für Aufnahme- und Verteillagen aus Vliesstoff und/oder Folie, aber auch für Saug/Speicherschichten stehen.

Um eine gute Benetzung von Polymeroberflächen und damit die gewünschte Hydrophilie zu erreichen, muß die Oberflächenenergie von Substraten aus beispielsweise Vlies oder Folie größer als jene des Benetzungsagens sein.

Demzufolge werden beispielsweise Polyolefine, gemäß ihrer unpolaren chemischen Struktur und einer Oberflächenenergie von Polypropylen (PP) ca. 30 mN/m nur unzureichend von Wasser benetzt. Es wäre dazu eine Oberflächenenergie von 72 mN/m erforderlich.

Zur Erhöhung der Oberflächenenergie sind aus dem Stand der Technik verschiedene Möglichkeiten bekannt, eine mehr oder weniger dauerhafte Hydrophilie bei Flüssigkeitstransportlagen zu erzeugen.

So zeigen die DE 196 45 380 A1 und EP 0410 485 B1 Möglichkeiten zur permanenten Hydrophilierung mittels des Auftrags von Avivagen auf die Oberfläche von derartigen Flüssigkeitstransportlagen. Erreicht wird die Permanenz im allgemeinen dabei zum einen durch hohen Avivage-Auftrag und zum anderen mittels schlechter Wasserlöslichkeit der eingesetzten Avivage-Systeme, damit die aufgebrachten Mengen nur schwer abgelöst werden können. Nachteilig bei dieser Arbeitsweise ist, dass der hydrophile Effekt mit wiederholter Flüssigkeitsbeladung, bedingt durch das Auswaschen der Avivage, nachlässt.

Eine andere Möglichkeit ist das Einbringen von hydrophilen Substanzen in die Schmelze der für der Herstellung der Flüssigkeitstransportlagen zum Einsatz kommenden Polymere. Derartige Verfahren werden in der DE 197 12 379 A1 oder 198 51 687 A1 beispielhaft gezeigt. Nachteilig ist hier die Tatsache, dass die eingebrachten Hydrophilierungsmittel nur in das Polymergefüge eingelagert, aber nicht chemisch verankert sind. Es wird hier ebenfalls ein Nachlassen der hydrophilen Effekte über die Zeit beobachtet.

Andere Arten der chemischen Modifizierung, beispielsweise der Pfropfpolymerisation aus DE 197 15 449 A1 vermeiden dies zwar, haben aber den Nachteil, dass die Modifizierung bereits bei der Polymerisation der Ausgangsrohstoffe wie Granulaten geschehen muss. Nachteilig, neben den hohen Einstandspreisen für derartige Materialien, ist die Tatsache, dass diese Granulate durch die Modifizierung negative Verarbeitungseigenschaften erfahren.

Werden Flüssigkeitstransportlagen aus Vliesstoff verwendet, ist auch eine Hydrophilierung mittels Bindemittelsystem denkbar. Ein derartiges Verfahren und Produkt ist in EP 0 842 650 A1 beschrieben. Nachteilig wirkt sich dabei die Tatsache aus, dass hier mit einem Verfahren gearbeitet wird, welches einen Verdampfungs-/Trocknungsgang mit nachgeschalteter Vernetzung des Binders beinhaltet. Diese Arbeitsweise ist energieintensiv und nur mit geringen Produktionsgeschwindigkeiten darstellbar.

Als Hauptnachteil aller genannten Methoden zur Erreichung einer permanenten Hydrophilie ist allerdings der Umstand zu nennen, dass die während des Gebrauchs durch Körperflüssigkeiten ausgewaschenen Anteile der Hydrophilierungsmittel, es handelt sich hierbei im weitesten Sinne um Tenside, eine Reduktion der Oberflächenspannung der durch sie dringenden Körperflüssigkeiten zur Folge hat.

Dieses Auswaschen der wasserlöslichen organischen Avivagen setzt die Oberflächenspannung $\sigma$ von Wasser ($\sigma \approx 72$ mN/m) stark herab.

Die Reduktion der Oberflächenspannung - sie kann teilweise bis zu 45% betragen - bewirkt darüber hinaus eine partielle Inaktivierung der zur Speicherung dieser Körperflüssigkeiten vorgesehenen nachfolgenden Saug-/ Speicherschichten.

Speziell betroffen davon sind sogenannte superabsorbierende Substanzen, welche durch Interaktion mit den ausgewaschenen Tensiden in ihrer Quellfähigkeit deutlich eingeschränkt werden. Damit nun trotzdem ein ausreichendes Speichervermögen erreicht wird, ist ein Mehreinsatz derartiger Speichersubstanzen notwendig, was andere Probleme wie beispielsweise Auspudern dieser oder ähnliches nach sich zieht.

Eine Aufstellung von Oberflächenspannungen wäßriger Lösungen nach einer Behandlung mit dem Stand der Technik entsprechenden hydrophoben, hydrophilen und permantent hydrophilen PP- und PET- Vlies- Materialien ist in nachstehender Tabelle 1 aufgelistet.

Tabelle 1:

| **Oberflächenspannungen wäßriger 0,9% NaCl-Lösung und deren Reduktion nach Passage von Vliesen aus unbehandelten PP und PET-Fasern** (Oberflächenspannung 0,9%NaCl-Lösung = 72mN/m) | | |
|---|---|---|
| **Material** | **Oberflächenspannung [mN/m]** | **Reduktion in %** |
| Vlies A, 100% PP, hydrophob, 2,2 dtex | 59,3 | 17,6 |
| Vlies B, 100% PP, hydrophob, 6,7 dtex | 60,5 | 16,0 |
| Vlies C, 100% PP perm.hydrophil, 6,7 dtex | 53,7 | 25,4 |
| Vlies D,100% PET, hydrophil, 5,3 dtex (50%), 12,0 dtex (50%) | 52,0 | 27,8 |
| Vlies E, 100% PET,perm.hydrophil 7,0 dtex | 42,1 | 41,5 |
| Vlies F,100% PET, perm.hydrophil 6,7 dtex | 50,8 | 29,4 |

Aufgabe der Erfindung war es daher, unter Vermeidung der genannten Nachteile des Standes der Technik, permanent hydrophile Flüssigkeitstransportlagen für Hygieneartikel bereitzustellen.

Die Aufgabe ist gemäß den Merkmalen der Ansprüche 1 und 2 gelöst. Sinnvolle Ausgestaltungen sind den Unteransprüchen 3 bis 9 aufgezeigt.

Der Erfindung benutzt die Idee, eine permanente Hydrophilierung nicht an den Rohmaterialien, sondern an fertigen Materialbahnen aus Vlies oder Folie vorzunehmen.

Die grundlegende Überlegung dabei war, diese Materialbahnen chemisch dergestalt zu verändern, dass in der Polymerstruktur stark polare Bereiche entstehen.

Die eigentliche Erfindung wird darin gesehen, dass auswaschbare Anteile der Materialbahn, welche die Oberflächenspannung von diese passierenden Flüssigkeiten reduzieren, durch die spezielle Oxifluorierung deaktiviert werden.

Als Messgröße dafür wurde der bereits vorab eingeführte Begriff der Reduktion der Oberflächenspannung verwendet.

Zur Ermittlung der Oberflächenspannung kam dabei eine auf der DIN 53914 basierende Methode zur Anwendung. In Abwandlung dieser wurde dabei als Messgerät ein digitales Plattentensiometer Typ K10 des Herstellers Krüss, verwendet. Von einer 0,9% Kochsalzlösung wurde zunächst der Ausgangswert der Oberflächenspannung $\sigma_{vorher}$ bei Raumtemperatur bestimmt . Danach wurden 0,15g der zu prüfenden Flüssigkeitstransportlage während 20 Minuten in der vorgenannten Lösung behandelt. Der Extrakt wurde anschließend wiederum bezüglich der Oberflächenspannung $\sigma_{nachher}$ untersucht.

Die Reduktion wird dann nach folgender Gleichung ermittelt:

$$\text{Reduktion (\%)} = \frac{(\sigma_{vorher} - \sigma_{nachher})}{\sigma_{vorher}} * 100\%$$

Der Effekt, dass durch Reaktion polyolefinischer Polymere mit Halogenen eine Verbesserung der Benetzungseigenschaften der aus den Polymeren erhaltenen Folien oder Faserstoffen und damit Zunahme der Hafteigenschaften erzielt werden kann, wurde grundlegend von Hänsel, Schulz, Koi in "Untersuchungen zur Oberflächen-modifizierung durch den Prozess der Gasphasenfluorierung", erschienen in COATING 7/1998, Seite 246-249 untersucht. Die Technik dazu wurde in "Oberflächen vorbehandeln mit Fluor" von Milker und Möller, erschienen in KUNSTSTOFFE 82 (1992), Seite 978-981 beschrieben. Auch aus dem Bereich der Batterieseparatoren sind ähnliche Verfahren bekannt, vergleiche hierzu DE 195 23 231 C1.

Die Reaktion von fluorhaltigen Gasgemischen (in der Regel Stickstoff/Fluor = 90% / 10%) mit Polymeren ist auf die ausgewiesene Reaktivität des Fluors ($F_2$) zurückzuführen. Dabei begründet sich die hohe Reaktionsfähigkeit des zweiatomigen Halogens zum einen auf den geringen Energieaufwand zur Spaltung der F — F-Bindung (Bindungsdissoziationsenergie $\Delta_D H = 158 \text{ kJ} \times \text{mol}^{-1}$ ) sowie den hohen, resultierenden mittleren Bindungsenergien (Energieabgabe $\Delta_{\bar{B}} H = 456 \text{ kJ} \times \text{mol}^{-1}$) bei der Formierung von C - F-Bindungen. Das Ergebnis der nach dem radikalischen Mechanismus verlaufenden, stark exothermen Substitution von Wasserstoff-Atomen (H) durch Fluor-Atome im Polypropylen wird anhand nachfolgender Reaktionsgleichung beispielhaft verdeutlicht.

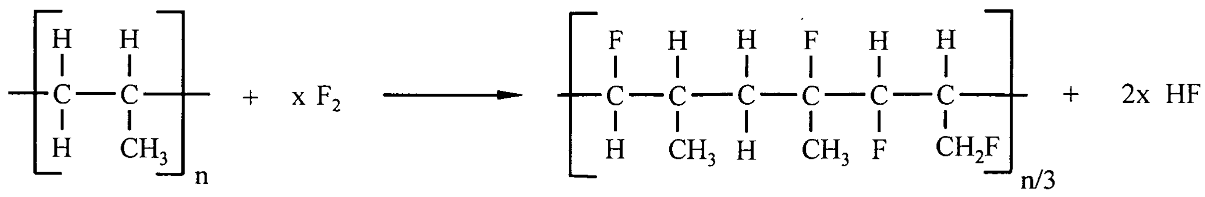

Polypropylen — partiell fluoriertes Polypropylen

Angemerkt sei, daß es sich bei den vorliegenden Prozeßen genau genommen um Oxifluorierungen handelt, da die Polymeroberfläche unmittelbar nach der $F_2$-Beaufschlagung eine Vielzahl von radikalischen Stellen aufweist, welche einen Angriff von Sauerstoffmolekülen ($O_2$) gestatten. Somit wird beispielsweise die Bildung von Carbonyl- (-CHO), Keto- (>CO), Carboxyl- (-COOH) und Carbonsäurefluorid (-COOF) - Gruppen ermöglicht. Das Auftreten des $O_2$ in der Reaktionskammer ist auf einen in der Regel nicht vollständig evakuierten Reaktor zurückzuführen.

Neben der Generierung einer chemisch aufgerauhten Polymeroberfläche infolge verschiedener Atomdurchmesser der beteiligten Atome H, O und F ist die Ursache für den Anstieg der Hydrophilie hauptsächlich in der Zunahme der Elektronegativitätsdifferenz $\Delta\chi$ der Bindungspartner der C — X-Bindung (X = H, O, F) zu sehen. Während $\Delta\chi_{C-H}$ der Kohlenstoff — Wasserstoff-Bindung einen Wert von 0,4 erreicht, ergibt sich für die Kohlenstoff — Sauerstoff-Bindung bereits $\Delta\chi_{C-O}$ = 1,4, für die Kohlenstoff — Fluor-Bindung gar ein Wert der Elektronegativitätsdifferenz von 1,9. Als eine Folge der somit veränderten Bindungssymmetrie zeigt sich eine merkliche Polaritätserhöhung der C — F(O)-Bindung mit negativem Ladungsschwerpunkt an den Hetero-Atomen. Das resultierende Dipolmoment des partiell oxifluorierten Olefins ergibt einen markanten Anstieg der Oberflächenenergie und ermöglicht hierdurch eine verbesserte Anlagerung von Wasser-Dipolmolekülen an das Polymer. Eine deutliche Erhöhung der Hydrophilie wird bei PP nach einer Substitution von ca. 20 at % der H-Atome durch F-Atome und einem O-Anteil von ebenfalls etwa 20 at% erzielt.

Neben Polyolefinen sind auch weitere Polymere wie Polyester (PET u.a.) oder Polyamide dem Verfahren zugänglich.

Zur Veranschaulichung der Erfindung sind nachfolgende Beispiele aufgeführt. Der Einfachheit halber sind diese auf Vliesstoffe bezogen, können aber mit jeder anderen als Flüssigkeitstransportlage verwendbaren Bahnenware wie Folien, Geweben, Gewirken oder dergleichen ausgeführt werden.

Handelsübliche Aufnahme-/ Verteilvliese aus hydrophobem PP (Vlies B aus Tabelle 1) und aus hydrophilem PET (Vlies D aus Tabelle 1) wurden den nachstehenden Behandlungen unterzogen. Dabei wurden die Verfahrensbedingungen wie F2-Konzentration und Reaktionszeit variiert. Auch wurde der Einfluss von diskontinuierlicher Arbeitsweise im Reaktor und kontinuierlicher Warenbewegung auf die erzielbare Hydrophilie untersucht. Im einzelnen wurden folgende Bedingungen dargestellt:

(a) Reaktor: 2% $F_2$, 120 sec. und 4% $F_2$, 180 sec.
(b) Kontinuierlich: 2% $F_2$ bei v = 10 m/min (ergibt 12 sec. Behandlungszeit)

Die Tabellen 2a und 2b beinhalten eine Aufstellung der Effekte bezüglich der Hydrophilie. Dargestellt sind zunächst Strike-Through-Zeiten und die Rewet-Werte der vorgenannten oxifluorierten PP- und PET-Vliese im Vergleich zur jeweiligen unbehandelten Qualität. In den Abbildungen 1 und 2 sind die Ergebnisse graphisch dargestellt.

## Tabelle 2 a: Vergleich der Strike Through-Zeiten und Rewet-Werte von Vlies B vor und nach verschiedenen $F_2$-Behandlungen

| ***Vlies B*** 100% PP (6,7 dtex), hydrophobes Vlies, thermobondiert, 60 g/m$^2$ | Strike through [sec.] Methode EDANA 150.4-99 Wiederholte Beaufschlagung | | | | | Rewet [g] Methode EDANA 151.2-99 Wiederholte Beaufschlagung | | | | |
|---|---|---|---|---|---|---|---|---|---|---|
| | 1. | 2. | 3. | 4. | 5. | 1. | 2. | 3. | 4. | 5. |
| **unbehandelt** | > 300 | | | | | — | | | | |
| **Reaktor** | | | | | | | | | | |
| 2% $F_2$ / 120 sec. | 6,5 | 4,8 | 4,2 | 4,1 | 4,0 | 0,09 | 0,08 | 0,08 | 0,08 | 0,10 |
| 4% $F_2$ / 180 sec. | 4,9 | 2,7 | 2,9 | 3,1 | 2,8 | 0,08 | 0,08 | 0,08 | 0,08 | 0,07 |
| **Kontinuierlich** | | | | | | | | | | |
| 2% $F_2$ / 10 m/min | 4,9 | 4,2 | 4,1 | 4,0 | 3,7 | 0,08 | 0,08 | 0,08 | 0,08 | 0,08 |

## Tabelle 2 b: Vergleich der Strike Through-Zeiten und Rewet-Werte von Vlies D vor und nach verschiedenen $F_2$-Behandlungen

| ***Vlies D*** 100% PET (50% 5,3 dtex; 50% 12,0 dtex), hydrophiles Vlies, thermobondiert, 50 g/m$^2$ | Strike through [sec.] Methode EDANA 150.4-99 Wiederholte Beaufschlagung | | | | | Rewet [g] Methode EDANA 151.2-99 Wiederholte Beaufschlagung | | | | |
|---|---|---|---|---|---|---|---|---|---|---|
| | 1. | 2. | 3. | 4. | 5. | 1. | 2. | 3. | 4. | 5. |
| **Unbehandelt** | 3,2 | 2,8 | 3,8 | 5,3 | 6,8 | 0,11 | 0,10 | 0,18 | 0,34 | 0,48 |
| **Reaktor** | | | | | | | | | | |
| 2% $F_2$ / 120 sec. | 2,1 | 2,5 | 2,8 | 3,0 | 3,2 | 0,09 | 0,08 | 0,08 | 0,08 | 0,08 |
| 4% $F_2$ / 180 sec. | 3,2 | 2,7 | 3,0 | 3,2 | 2,9 | 0,09 | 0,08 | 0,09 | 0,09 | 0,09 |
| **Kontinuierlich** | | | | | | | | | | |
| 2% $F_2$ / 10m/min | 2,0 | 2,2 | 2,3 | 2,5 | 2,6 | 0,10 | 0,11 | 0,10 | 0,10 | 0,10 |

Während sich also bei den nach dem Stand der Technik mit Avivage ausgerüsteten Materialien die Strike-Through-Zeiten bei wiederholter Beaufschlagung - infolge des Abwaschens der Tenside - sukzessive erhöhen, werden bei den mit oxifluorierten Materialien ab der zweiten Flüssigkeitsaufgabe nahezu die gleichen Zeiten (PP: $t < 5$ sec., PET: $t < 3$ sec.) detektiert. Dies spricht für die Permanenz der Hydrophilierung, wie sie bei Materialien nach dem Stand der Technik nicht beobachtet werden kann. Sie unterstreicht weiterhin den Einbau von F- und O-Atomen sowie deren feste Bindung am Polymer.

Eine Aufstellung der Werte für die Oberflächenspannung von 0,9%iger Kochsalzlösung vor und nach deren Kontakt mit den Vliesstoffen B und D ist in Tabelle 3 wiedergegeben. Die entsprechenden Grafiken für die Reduktion

der Oberflächenspannung zeigen die Abbildungen 3 und 4.

| **Tabelle 3: Oberflächenspannungen wäßriger Lösungen nach deren Wechselwirkung mit unbehandelten bzw. gasphasenfluorierten PP und PET- Vliesstoffen** | | |
|---|---|---|
| | **Oberflächenspannung [mN/m] / Reduktion [%] PP (Vlies B)** | **Oberflächenspannung [mN/m] / Reduktion [%] PET (Vlies D)** |
| physiologische NaCl-Lösung | größer 72,0 | |
| unbehandelt | 60,5 / 16,0 | 52,0 / 27,8 |
| 2% $F_2$, 120 sec. (Reaktor) | 70,2 / 2,5 | 67,2 / 6,7 |
| 4% $F_2$, 180 sec. (Reaktor) | 69,7 / 3,2 | 68,6 / 4,7 |
| 2% $F_2$, 10 m/min, (Bahnware) | 72,0 / 0,0 | 68,2 / 5,3 |

Infolge der nur an der Oberfläche des Polymers auftretenden Substitutionen H ↔ F, O tritt keine Schädung der untersuchten Materialien im Zuge der Oxifluorierung auf. Die in Tabelle 4 aufgeführten mechanisch-technischen Daten des unbehandelten bzw. oxifluorierten PET-Vliesstoffes D veranschaulichen dies.

Tabelle 4 :

| **mechanisch-technische Daten von Vlies D vor und nach der Gasphasenfluorierung** | | | | |
|---|---|---|---|---|
| **a) Querrichtung** | | | | |
| | Unbehandelt | 2% Fluor; 180 sec. | 4% Fluor; 180 sec. | 2% Fluor; 10m/min; |
| $F_{max}$ [N/50 mm] | 3,9 | 3,8 | 5,5 | 5,2 |
| $F_{max}$ - Dehnung [%] | 27,8 | 33,5 | 26,2 | 23,2 |
| Bruchdehnung [%] | 35,3 | 47,3 | 32,6 | 30,7 |
| 5N-Dehnung [%] | 0,0 | 0,0 | 18,1 | 18,0 |
| **b) Längsrichtung** | | | | |
| | Unbehandelt | 2% Fluor; 180 sec. | 4% Fluor; 180 sec. | 2% Fluor; 10m/min |
| $F_{max}$ [N/50 mm] | 19,6 | 21,8 | 29,0 | 22,0 |
| $F_{max}$ - Dehnung [%] | 5,8 | 7,5 | 8,0 | 6,4 |
| Bruchdehnung [%] | 7,2 | 9,7 | 10,8 | 7,8 |
| 5N-Dehnung [%] | 1,6 | 1,6 | 1,4 | 1,5 |

Sowohl die behandelten als auch die unbehandelten Vliesstoffe wurden mittels Röntgen-Photoelektronen-Spektroskopie (XPS, auch ESCA = Elektronenspektroskopie für die chemische Analyse), Fourier-Transform-Infrarot-Spektroskopie (FTIR) und zum Teil auch mit Hilfe der Energiedispersiven Röntgenmikroanalyse (EDX) im Rasterelektronenmikroskop charakterisiert. Zur Untersuchung der oberflächennahen Schichten wurde bei den IR-spektroskopischen Untersuchungen die Methode der abgeschwächten Totalreflexion (ATR) verwendet.

Durch oberflächensensitive XPS-Untersuchungen mit einer Analysetiefe von ca. 5nm konnte die chemische Zusammensetzung der oxifluorierten PP- und PET-Oberflächen zweifelsfrei analysiert werden. Die Tabellen 5a und 5b zeigen die Elementverteilung in Atomprozenten (at%) von drei Oxifluorierungsvarianten im Vergleich zur jeweiligen unbehandelten Probe.

Tabelle 5a:

| Oberflächenzusammensetzung mittels XPS des PP-Vlieses B, at% (ohne Wasserstoff) | | | | | |
|---|---|---|---|---|---|
| **Probe** | **C** | **N** | **O** | **F** | **Si** |
| PP - unbehandelt | 87,8 | 3,5 | 6,6 | - | 2,1 |
| PP — 2% $F_2$, 120 sec. (Reaktor) | 63,4 | 0,8 | 17,1 | 18,3 | 0,5 |
| PP - 4% $F_2$, 180 sec. (Reaktor) | 58,8 | 1,6 | 17,4 | 21,9 | 0,4 |
| PP - 2% $F_2$, 10 m/min (kontinuierlich) | 61,7 | 0,7 | 16,6 | 20,7 | 0,3 |

Tabelle 5b:

| Oberflächenzusammensetzung mittels XPS des PET-Vlieses D, at% (ohne Wasserstoff) | | | | | |
|---|---|---|---|---|---|
| **Probe** | **C** | **N** | **O** | **F** | **Si** |
| PET - unbehandelt | 71,8 | - | 25,5 | - | 2,7 |
| PET - 2% $F_2$, 120 sec. (Reaktor) | 62,0 | 1,1 | 25,3 | 11,4 | 0,3 |
| PET- 4% $F_2$, 180 sec. (Reaktor) | 62,6 | 0,4* | 24,2 | 12,3 | 0,5 |
| PET - 2% $F_2$, 10 m/min (kontinuierlich) | 59,5 | 0,8 | 25,8 | 13,5 | 0,4 |

Die Fluorgehalte der behandelten PP-Vliese liegen bei ca. 20 at%, die der PET-Qualitäten bei ca. 12 at% und entsprechen somit den zu erwartenden Werten für die einzelnen Polymere. Fast ausschließlich treten dabei C — F-Bindungen auf, wohingegen C — $F_2$-Bindungsanteile vernachlässigbar sind. Während beim PP als Folge der Oxifluorierung ein Anstieg der Sauerstoffgehalte auf ca. 17 at% feststellbar ist, zeigen hingegen die Sauerstoffanteile bei den PET-Proben kaum eine Veränderung. Der maximale Fluor- und Sauerstoff-Gehalt kann auch bei längerer Einwirkzeit und/oder höherer $F_2$-Konzentrationen, beispielsweise 4% $F_2$, 180 sec. nicht gesteigert werden, so daß das Verfahren bereits bei niedrigem Verbrauch des Prozeßgases $F_2$ und höheren Fahrgeschwindigenkeiten die gewünschten Effekte ermöglicht.

Während die in Tabelle 5 genannten Reaktionsbedingungen nur eine Fluorierung der obersten Atomlagen gestatten und somit kein Nachweis der Fluorierung mittels FTIR-Spektroskopie bzw. EDX gelingt, ist dagegen bei einer $F_2$-Konzentration von 10% und einer Reaktionszeit von 180 sec. die Fluorierung mit diesen Methoden eindeutig nachweisbar. So zeigt das IR-Spektrum des fluorierten PP durch das Auftreten der C — F-Bande ($\bar{\upsilon}$ = 1098 $cm^{-1}$) deutlich das Vorliegen von C — F-Bindungen an. Desweiteren unterstreicht die weniger intensive Bande bei $\bar{\upsilon}$ = 1715 $cm^{-1}$ die Bildung von Carbonylgruppen (C = O) im Zuge des Oxifluorierungsprozesses. Die "harten" Fluorierungsbedingungen (10% $F_2$, 180 sec.) ermöglichen demzufolge nicht nur eine Generierung von C — F-Bindungen in den obersten Atomschichten, sondern auch jene in tieferliegenden Zonen des Polymers. Dies kann auch durch EDX-Messungen belegt werden. So werden beispielsweise beim PP immerhin fast 7 at% $F_2$ in einer Analysetiefe bis ca. 3 $\mu$m nachgewiesen.

Interessant ist desweiteren ein Blick auf die "Nebenbestandteile" N, Si und O der PP-Proben. Diese Elemente sind beim unbehandelten Vlies B des Ausführungsbeispiels als Bestandteil der Avivage eindeutig nachweisbar, ihr Anteil vermindert sich aber nach dem Prozeß der Gasphasenfluorierung stark (Ausnahme: Sauerstoff). N und Si sind aber noch quantifizierbar. Dies beweist den Abbau der Avivage zu niedermolekularen Produkten wie beispielsweise HF, $CF_4$, $CO_2$.

Verarbeitungshilfen, beispielsweise in Form von als Avivagen aufgebrachten Antistatika und Hydrophilierungsmitteln, sind also bei erfindungsgemäßen Flüssigkeitstransportlagen zerstört. Wie Tabelle 3 zeigt, beeinflussen sie daher nach Oxifluorierung die Oberflächenspannung von sie durchdringenden Flüssigkeiten nicht mehr.

Betrachtet man die Abbildung 2, so kann man erkennen, dass bei Materialien, welche nach dem Stand der Technik gefertigt sind, die Hydrophilie, ausgedrückt als Strike-Through, von Beaufschlagung zu Beaufschlagung abnimmt.

Erfindungsgemäßes Material hingegen ändert auch bei einer Vielzahl von Beaufschlagungen seinen hydrophilen Charakter nicht, es kann hier wirklich von einer Permanenz der Hydrophilie gesprochen werden.

Auch ein Blick auf die Tabelle 3 und die Abbildungen 3 und 4 unterstreicht, dass sich das erfindungsgemäße Material deutlich vom Stand der Technik abhebt. Der Kontakt von Körperflüssigkeiten, hier ersatzweise physiologischer Kochsalzlösung, mit den unbehandelten Varianten der Vliese B und D zeigt eine deutliche Reduktion der Oberflächenspannung dieser Flüssigkeiten von bis zu 27%.

Flüssigkeiten, welche in Kontakt mit den behandelten, erfindungsgemäßen Ausführungen von Vlies B und D standen, zeigen diese Reduktion nicht mehr. Dies bedeutet, dass aus den Vliesen keine Substanzen mehr ausgewaschen werden, welche die Leistung von in Hygienartikeln enthaltenen, flüssigkeitsspeichernden Komponenten, beispielsweise Superabsorbern, beeinträchtigen.

Eine erfindungsgemäße Flüssigkeitstransportlage kann daher in allen flüssigkeitsleitenden oder -speichernden Schichten eines Hygieneartikel vorteilhaft verwendet werden, da sie sich neutral gegenüber anderen funktionalen Komponenten verhält.

**Patentansprüche**

1. Verwendung einer partiell oxifluorierten, aus thermoplastischen Polymeren bestehenden Schicht als permanent hydrophile Flüssigkeitstransportlage in Hygieneprodukten,
**dadurch gekennzeichnet,**
**dass** der Gehalt an Fluor dieser Schicht 25 Atomprozent nicht übersteigt.

2. Permanent hydrophile Flüssigkeitstransportlage für Hygieneprodukte unter Verwendung thermoplastischer Polymere,
**dadurch gekennzeichnet**
**dass** die Flüssigkeitstransportlage aus oxifluorierten, oberflächenmodifizierten Polymeren mit maximal 25 Atomprozent Fluorgehalt besteht.

3. Permanent hydrophile Flüssigkeitstransportlage nach Anspruch 1 oder 2,
**dadurch gekennzeichnet,**
**dass** diese aus thermoplastischen Polymeren aus der Reihe der Polyester besteht.

4. Permanent hydrophile Flüssigkeitstransportlage nach Anspruch 1 oder 2,
**dadurch gekennzeichnet,**
**dass** diese aus thermoplastischen Polymeren aus der Reihe der Polyolefine besteht.

5. Permanent hydrophile Flüssigkeitstransportlage nach Anspruch 1 oder 2,
**dadurch gekennzeichnet,**
**dass** diese aus thermoplastischen Polymeren aus der Reihe der Polyamide besteht.

6. Permanent hydrophile Flüssigkeitstransportlage nach Anspruch 1 oder 2,
**dadurch gekennzeichnet,**
**dass** diese aus einer Folie besteht.

7. Permanent hydrophile Flüssigkeitstransportlage nach Anspruch 1 oder 2,
**dadurch gekennzeichnet,**
**dass** diese aus einem Vlies besteht.

8. Permanent hydrophile Flüssigkeitstransportlage nach Anspruch 1 oder 2,
**dadurch gekennzeichnet,**
**dass** diese ein Flächengewicht zwischen 10 und 200 g/qm aufweist.

9. Permanent hydrophile Flüssigkeitstransportlage nach Anspruch 1 oder 2,
**dadurch gekennzeichnet,**
**dass** diese eine Dicke zwischen 0,01 und 5,0 mm aufweist.

Abbildung 1: Strike Through-Zeiten von Vlies B nach wiederholter Beaufschlagung

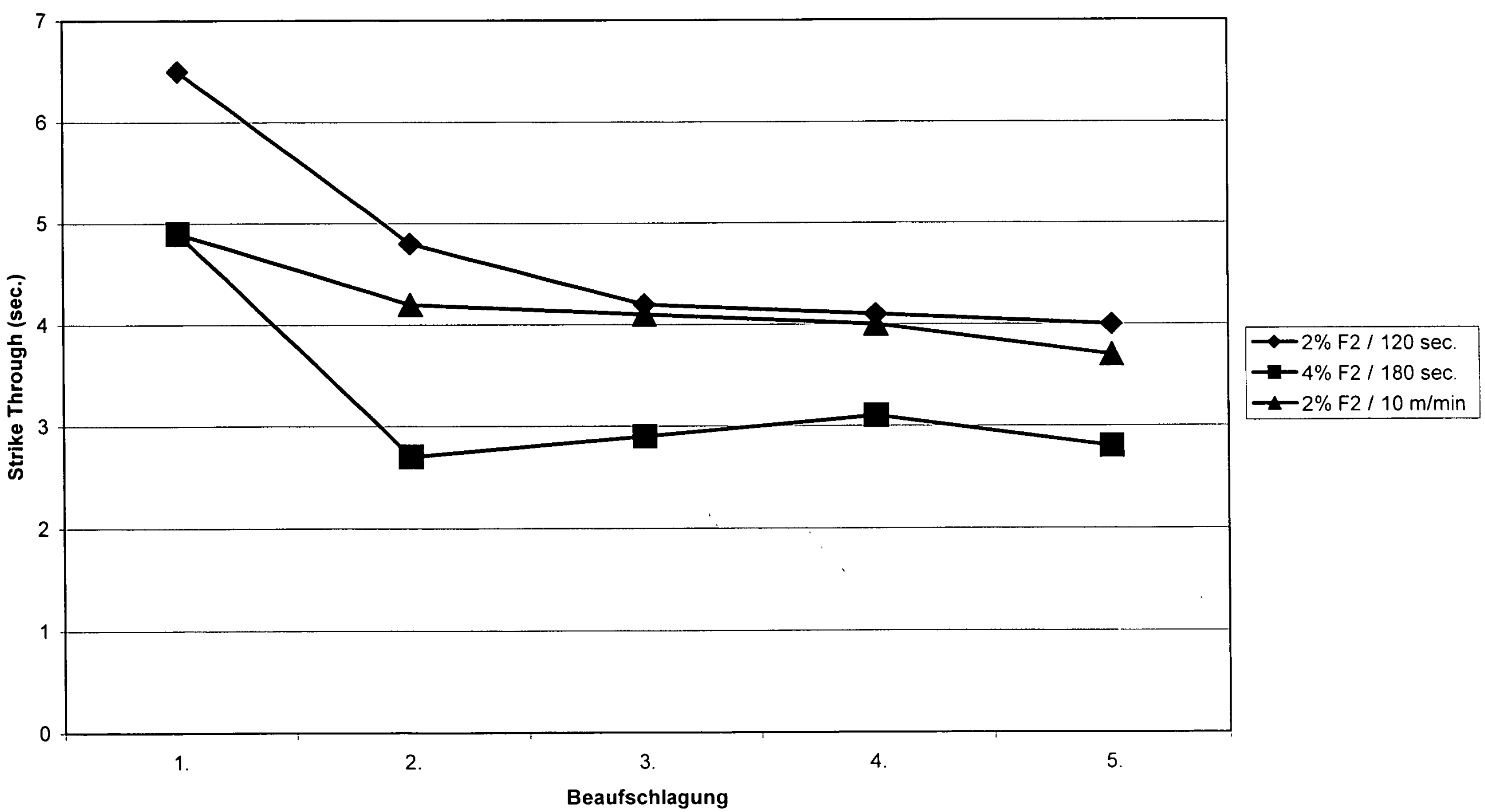


BAG; E-16-01-ZeichnungenEing;Abbildung 1    10.09.01

Abbildung 2: Strike Through von Vlies D bei wiederholter Beaufschlagung

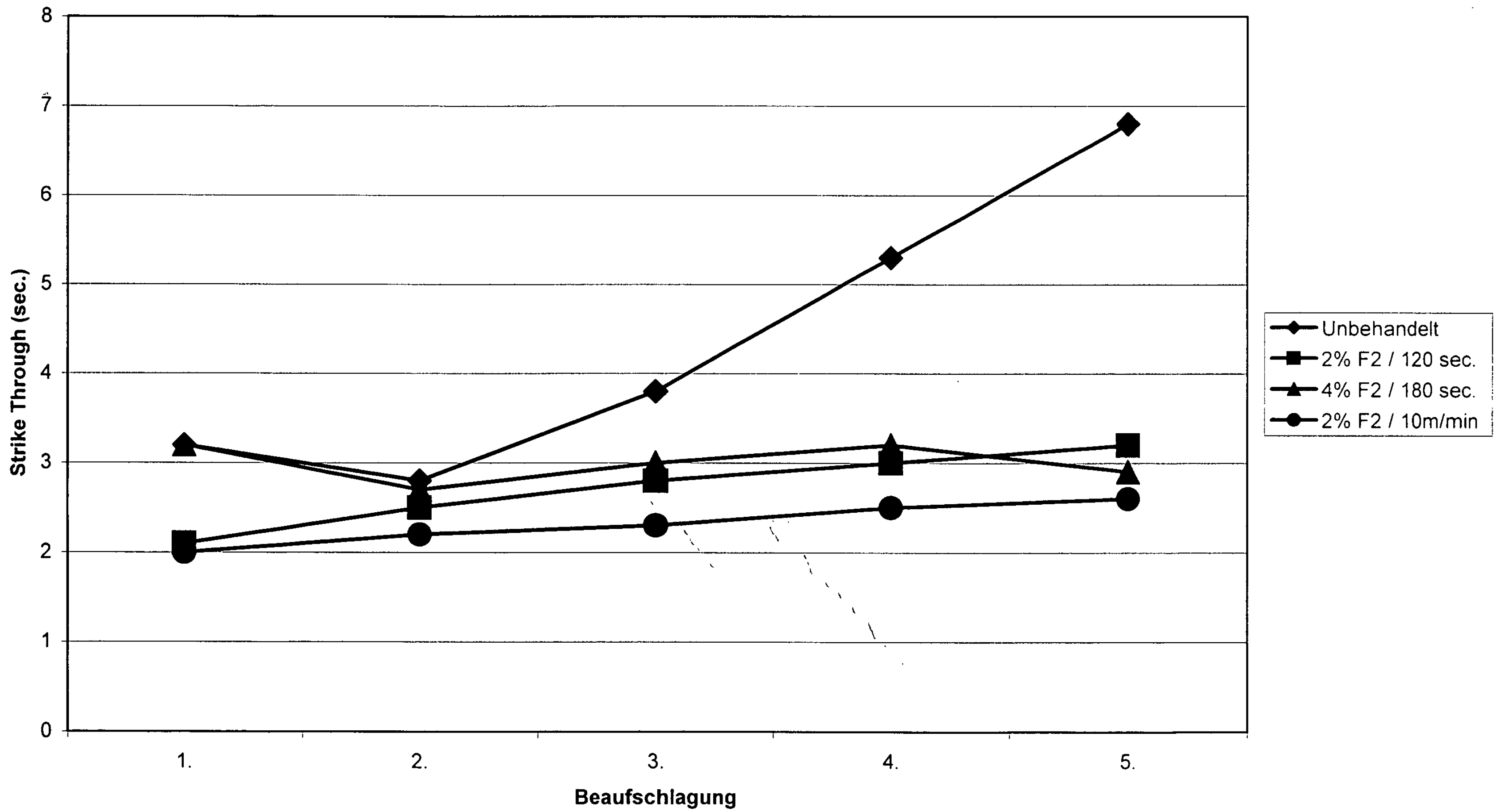


BAG; E-16-01-ZeichnungenEing;Abbildung 2 10.09.01

Abbildung 3: Reduktion der Oberflächenspannung von 0,9%NaCl-Lösung nach Kontakt mit Vlies B

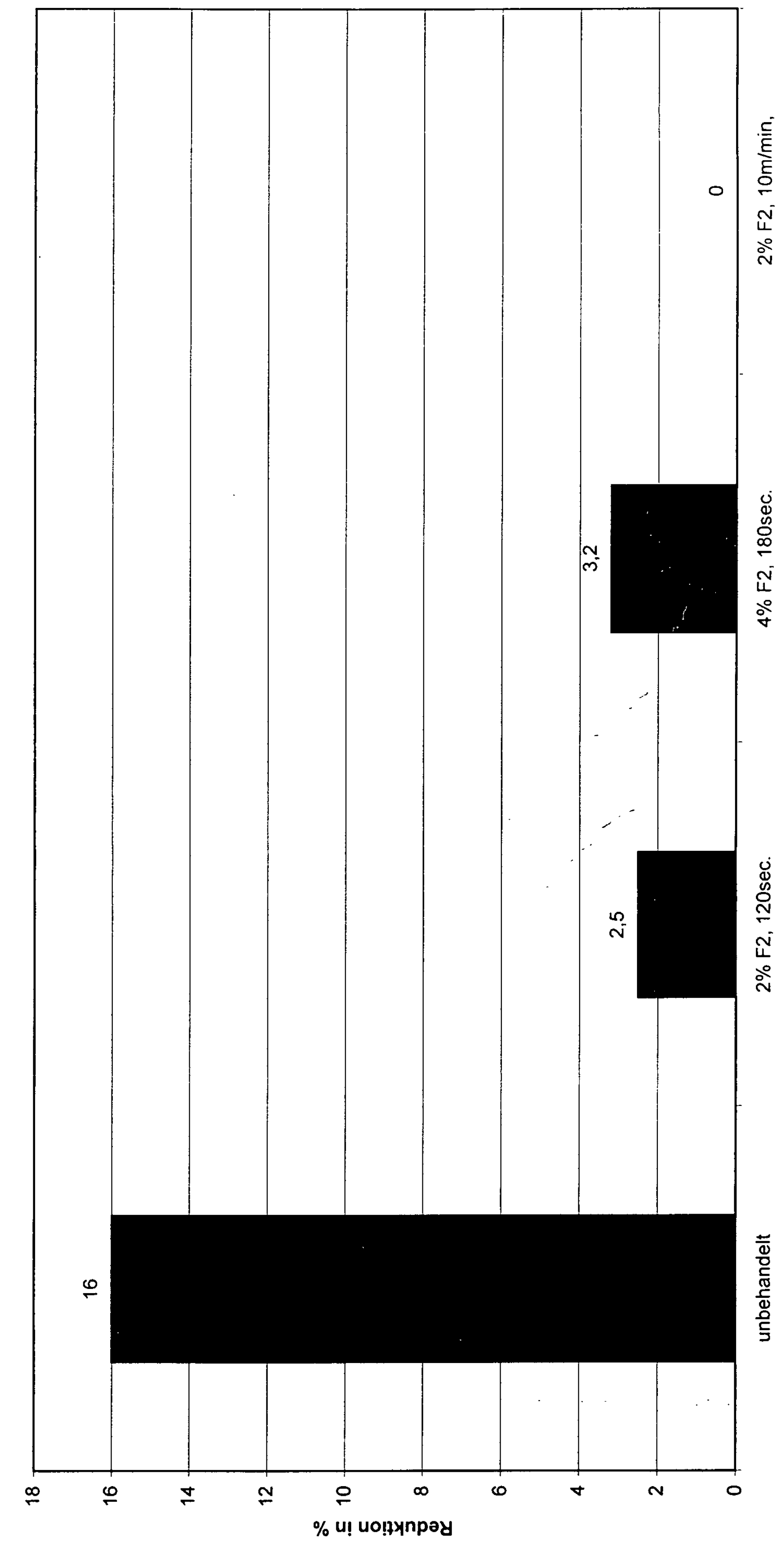


BAG; E-16-01-ZeichnungenEing;Abbildung3

10.09.01

**Abbildung 4: Reduktion der Oberflächenspannung von 0,9%NaCl-Lösung nach Kontakt mit Vlies D**

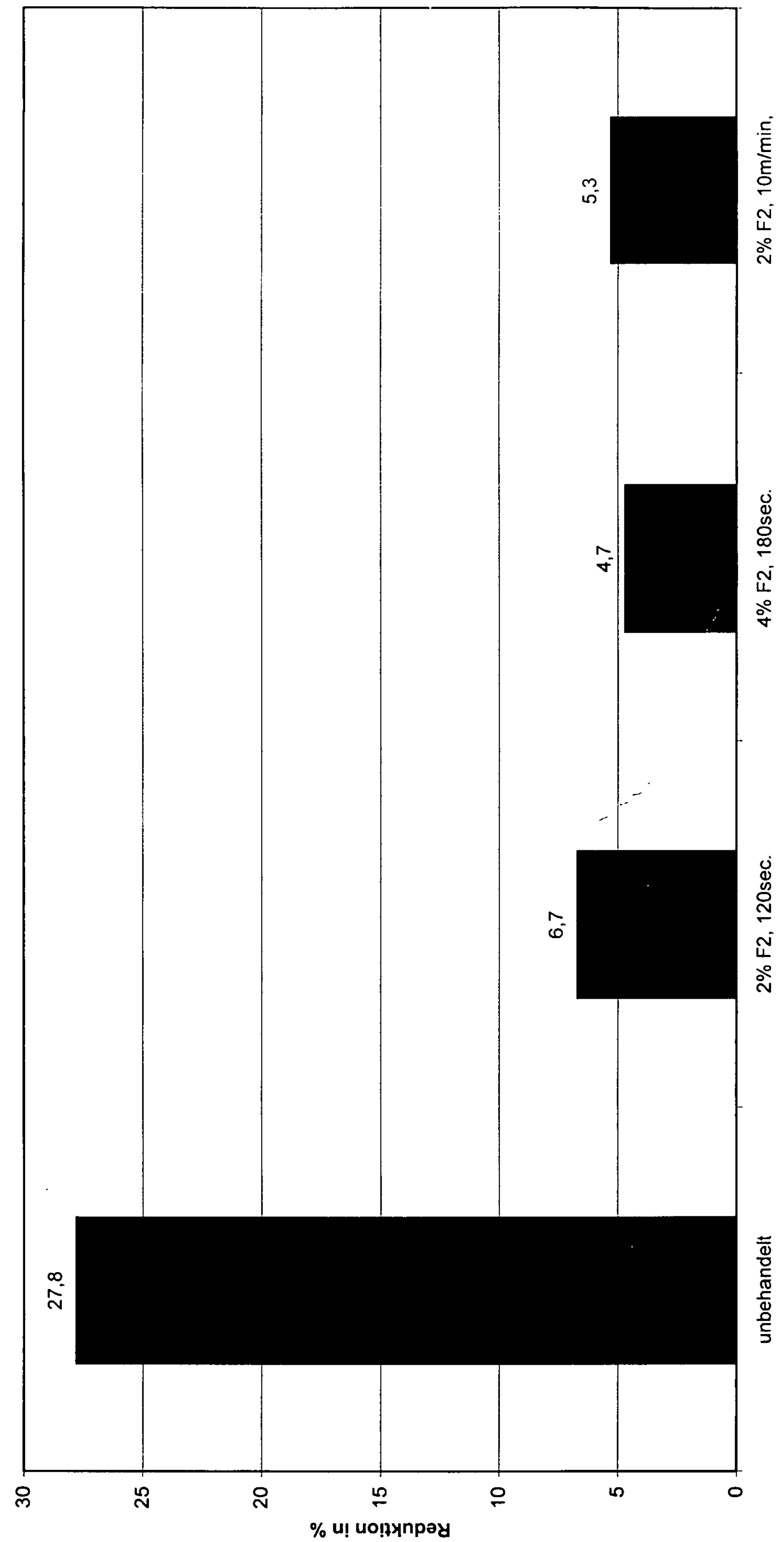


BAG; E-16-01-ZeichnungenEing;Abbildung 4

10.09.01

Europäisches Patentamt

# EUROPÄISCHER RECHERCHENBERICHT

Nummer der Anmeldung
EP 02 01 9830

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 5 700 254 A (MCDOWALL DEBRA JEAN ET AL) 23. Dezember 1997 (1997-12-23)<br>* Spalte 10, Zeile 6 - Spalte 11, Zeile 4 * | 1-9 | A61L15/26<br>A61F13/15 |
| A | US 5 531 728 A (LASH GLEN R)<br>2. Juli 1996 (1996-07-02)<br>* Spalte 3, Zeile 33-41 *<br>* Spalte 4, Zeile 14-18 *<br>* Spalte 6, Zeile 31-42,63-67 *<br>* Spalte 7, Zeile 1-16 *<br>* Spalte 16, Zeile 13-21 *<br>* Spalte 17, Zeile 11-14 * | 1-9 | |
| A | US 5 695 487 A (JAMESON LEE KIRBY ET AL)<br>9. Dezember 1997 (1997-12-09)<br>* Spalte 4, Zeile 22-35 * | 1-9 | |
| A | WO 91 02551 A (SMITH & NEPHEW)<br>7. März 1991 (1991-03-07)<br>* Ansprüche * | 1,2 | **RECHERCHIERTE SACHGEBIETE (Int.Cl.7)** |
| A | US 5 300 357 A (GARDINER ROBERT A)<br>5. April 1994 (1994-04-05)<br>* Spalte 3, Zeile 43-46 *<br>* Spalte 4, Zeile 31-59 *<br>* Spalte 7, Zeile 41-55 * | 2 | A61L<br>A61F<br>A61K |
| A,D | DE 197 12 379 A (HENKEL KGAA)<br>1. Oktober 1998 (1998-10-01)<br>* Zusammenfassung * | 2 | |
| A,D | DE 196 45 380 A (SCHILL & SEILACHER)<br>7. Mai 1998 (1998-05-07)<br>* das ganze Dokument * | 1-9 | |
| | -/-- | | |

Der vorliegende Recherchenbericht wurde für alle Patentansprüche erstellt

| Recherchenort | Abschlußdatum der Recherche | Prüfer |
|---|---|---|
| DEN HAAG | 19. Dezember 2002 | Böhm, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 02 01 9830

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | DE 41 31 746 A (HOECHST AG)<br>25. März 1993 (1993-03-25)<br>* Zusammenfassung * | 2 | |
| A | DE 42 43 995 A (GORE W L & ASS GMBH)<br>30. Juni 1994 (1994-06-30)<br>* das ganze Dokument * | 1-9 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 19. Dezember 2002 | Böhm, I |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 01 9830

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-12-2002

| Im Recherchenbericht angeführtes Patentdokument | Datum der Veröffentlichung | Mitglied(er) der Patentfamilie | Datum der Veröffentlichung |
|---|---|---|---|
| US 5700254 A | 23-12-1997 | AU 695130 B2 | 06-08-1998 |
| | | AU 1619295 A | 12-10-1995 |
| | | BR 9501235 A | 07-11-1995 |
| | | CA 2129210 A1 | 01-10-1995 |
| | | DE 69528484 D1 | 14-11-2002 |
| | | EP 0674891 A2 | 04-10-1995 |
| | | JP 7275293 A | 24-10-1995 |
| | | US 5876388 A | 02-03-1999 |
| | | ZA 9501878 A | 11-12-1995 |
| US 5531728 A | 02-07-1996 | US 5360420 A | 01-11-1994 |
| | | AT 158162 T | 15-10-1997 |
| | | AU 658361 B2 | 13-04-1995 |
| | | AU 7183791 A | 21-08-1991 |
| | | BR 9105936 A | 20-10-1992 |
| | | CA 2069443 A1 | 24-07-1991 |
| | | CN 1053544 A ,B | 07-08-1991 |
| | | CS 9100136 A2 | 13-08-1991 |
| | | CZ 290313 B6 | 17-07-2002 |
| | | CZ 289768 B6 | 17-04-2002 |
| | | DE 69127689 D1 | 23-10-1997 |
| | | DE 69127689 T2 | 15-01-1998 |
| | | DK 512010 T3 | 13-10-1997 |
| | | EG 19130 A | 30-07-1994 |
| | | EP 0512010 A1 | 11-11-1992 |
| | | ES 2106775 T3 | 16-11-1997 |
| | | FI 923341 A | 22-07-1992 |
| | | GR 3024773 T3 | 31-12-1997 |
| | | HK 1003027 A1 | 30-09-1998 |
| | | HU 63760 A2 | 28-10-1993 |
| | | HU 215911 B | 29-03-1999 |
| | | IE 910224 A1 | 31-07-1991 |
| | | JP 5503446 T | 10-06-1993 |
| | | NZ 236854 A | 27-09-1994 |
| | | PL 288812 A1 | 27-01-1992 |
| | | PL 168533 B1 | 29-02-1996 |
| | | PT 96544 A ,B | 15-10-1991 |
| | | SG 72670 A1 | 23-05-2000 |
| | | RU 2090170 C1 | 20-09-1997 |
| | | TR 27249 A | 21-12-1994 |
| | | WO 9111163 A1 | 08-08-1991 |
| US 5695487 A | 09-12-1997 | AU 685995 B2 | 29-01-1998 |
| | | AU 3202695 A | 27-03-1996 |
| | | BR 9508897 A | 06-01-1998 |
| | | CA 2199535 A1 | 14-03-1996 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 02 01 9830

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-12-2002

| Im Recherchenbericht angeführtes Patentdokument | Datum der Veröffentlichung | Mitglied(er) der Patentfamilie | Datum der Veröffentlichung |
|---|---|---|---|
| US 5695487 A | | DE 29522211 U1 | 17-08-2000 |
| | | DE 69515492 D1 | 13-04-2000 |
| | | DE 69515492 T2 | 11-07-2002 |
| | | EP 0782428 A2 | 09-07-1997 |
| | | ES 2142488 T3 | 16-04-2000 |
| | | WO 9607384 A2 | 14-03-1996 |
| | | ZA 9507022 A | 26-03-1996 |
| WO 9102551 A | 07-03-1991 | AT 135590 T | 15-04-1996 |
| | | AU 632158 B2 | 17-12-1992 |
| | | AU 6154690 A | 03-04-1991 |
| | | CA 2050940 A1 | 16-02-1991 |
| | | DE 69026091 D1 | 25-04-1996 |
| | | DE 69026091 T2 | 22-08-1996 |
| | | EP 0487566 A1 | 03-06-1992 |
| | | WO 9102551 A1 | 07-03-1991 |
| | | GB 2248554 A ,B | 15-04-1992 |
| | | JP 4506913 T | 03-12-1992 |
| | | US 5380260 A | 10-01-1995 |
| US 5300357 A | 05-04-1994 | US 5244951 A | 14-09-1993 |
| | | AU 648887 B2 | 05-05-1994 |
| | | AU 1497092 A | 05-11-1992 |
| | | CA 2066012 A1 | 03-11-1992 |
| | | DE 69221770 D1 | 02-10-1997 |
| | | DE 69221770 T2 | 26-02-1998 |
| | | EP 0516271 A1 | 02-12-1992 |
| | | JP 3135978 B2 | 19-02-2001 |
| | | JP 5186907 A | 27-07-1993 |
| | | KR 230711 B1 | 15-11-1999 |
| DE 19712379 A | 01-10-1998 | DE 19712379 A1 | 01-10-1998 |
| | | WO 9842898 A1 | 01-10-1998 |
| DE 19645380 A | 07-05-1998 | DE 19645380 A1 | 07-05-1998 |
| | | EP 0839947 A2 | 06-05-1998 |
| | | US 6008145 A | 28-12-1999 |
| DE 4131746 A | 25-03-1993 | DE 4131746 A1 | 25-03-1993 |
| DE 4243995 A | 30-06-1994 | DE 4243995 A1 | 30-06-1994 |
| | | DE 59308720 D1 | 06-08-1998 |
| | | EP 0603463 A2 | 29-06-1994 |
| | | JP 6298976 A | 25-10-1994 |
| | | US 5540837 A | 30-07-1996 |
| | | US 5630941 A | 20-05-1997 |

EPO FORM P0461

Für nähere Einzelheiten zu diesem Anhang : siehe Amtsblatt des Europäischen Patentamts, Nr.12/82